(19) Europäisches Patentamt
European Patent Office
Office européen des brevets

(11) Publication number: **0 233 117 B1**

(12) **EUROPEAN PATENT SPECIFICATION**

(45) Date of publication of patent specification: **29.01.92**

(51) Int. Cl.[5]: **C07C 323/04**, C07C 319/20

(21) Application number: **87400235.5**

(22) Date of filing: **03.02.87**

(54) **Preparation of 4-carboalkoxy-1,3-cyclohexanedione type compounds.**

(30) Priority: **03.02.86 US 825010**

(43) Date of publication of application:
**19.08.87 Bulletin 87/34**

(45) Publication of the grant of the patent:
**29.01.92 Bulletin 92/05**

(84) Designated Contracting States:
**CH DE FR GB LI**

(56) References cited:
**EP-A- 0 124 041**
**EP-A- 0 142 741**

(73) Proprietor: **EASTMAN KODAK COMPANY (a New Jersey corporation)**
**343 State Street**
**Rochester New York 14650(US)**

(72) Inventor: **McCombs, Charles Allan c/o Eastman Kodak Company**
**Patent Department 343 State Street**
**Rochester New York 14650(US)**

(74) Representative: **Parent, Yves et al**
**Kodak-Pathé Département Brevets et Licences Centre de Recherches et de Technologie Zone Industrielle**
**F-71102 Chalon-sur-Saône Cédex(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention concerns a novel and markedly improved process for the preparation of 4-carboalkoxy-1-,3-cyclohexanedione type compounds. The process is especially characterized by improved yields.

The invention more particularly concerns the preparation of certain 4-carboalkoxy-5-[2-(alkylthio)-alkyl]-1,3-cyclohexanedione compounds which are useful as intermediates in the preparation of herbicides according to and such as those disclosed in U.K. Patent 2,090,246.

In accordance with the present invention, an acetoacetic ester of the formula $R\text{-}OOCCH_2COCH_3$ is reacted with an unsaturated ester of the formula $R^1R^2C=CHCOOR^3$ in the presence of basic catalyst such as the highly preferred ethanolic sodium ethoxide catalyst to give a compound (I) of the formula

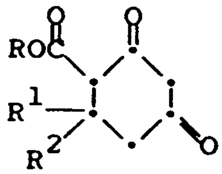

(I)

and the alcohol by-product $HOR^3$. By known hydrolysis and decarboxylation procedures, compound (I) is convertible to compound (II) of the formula

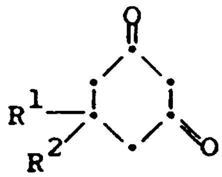

(II)

The useful basic catalysts include the groups IA and IIA metal alkoxides of $C_1$-$6_6$ aliphatic alcohols, or the hydroxides of these metals. Optionally, the basic catalyst can be prepared in situ by reacting metal with a $C_1$-$6_6$ alcohol. The same alcohol is also used as a solvent for the reaction.

In the above formulas : R is alkyl of 1-6 carbons ; $R^1$ and $R^2$ are each selected independently from H, alkyl, cycloalkyl, alkyl substituted cycloalkyl, aryl, substituted aryl, heterocyclic, or substituted heterocyclic, with the provision that at least one of $R^1$ and $R^2$ is an alkylthioalkyl group, wherein the alkyl groups or moieties are 1-18 carbons, preferably 1-6 carbons; and $R^3$ is alkyl of 1-6 carbons or cycloalkyl.

In the cyclization step of the present process, the preferred molar ratio of basic catalyst to total moles of reactants is from about 0.10 to about 10.0, most preferably from about 1 to about 4, the molar ratio of acetoacetic ester to unsaturated ester is from about 0.8 to about 1.5, preferably from about 1.0 to about 1.1, the weight ratio of total reactants to the $C_1$-$C_6$ alcohol is from about 0.01 to about 1.2, preferably from about 0.08 to about 1.0, the reaction temperature is from about 22°C to about 88°C, preferably from about 70°C to about 78°C, and the reaction period preferably is from about 5 to about 36, and most preferably from about 10 to about 24 hours.

The following table gives specific examples of the above R, $R^1$, $R^2$, and $R^3$ substituents. In the table each X substituent is in any ring position and is selected independently from H, alkyl of 1-6 carbons, alkoxy of 1-6 carbons, and halogen.

## TABLE

| $R^3$ | R |
|---|---|
| $CH_3$ | $CH_3$ |
| $C_2H_5$ | $C_2H_5$ |
| $C_3H_7$ | $C_3H_7$ |
| $C_4H_9$ | $C_4H_9$ |
| $C_5H_{11}$ | $C_5H_{11}$ |
| $C_6H_{13}$ | $C_6H_{13}$ |
| $C_6H_{11}$ | |

$R^1$ or $R^2$

$-H$, $-CH_3$, $-C_2H_5$, $-C_4H_9$, $-C_6H_{13}$,

$-C_8H_{17}$, $-C_{10}H_{21}$, $-(CH_2)_{17}CH_3$, 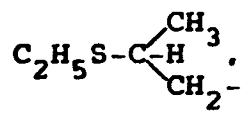 ,

$-C_6H_{11}$, $-C_6H_5$, 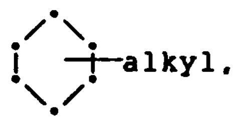 , 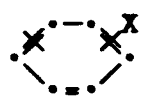

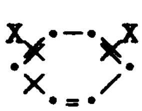 , 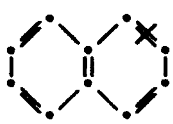 , 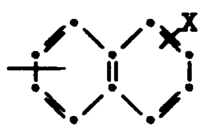 , 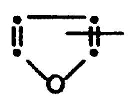 ,

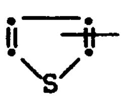 , 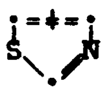 , 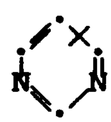 , 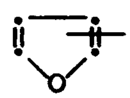 , 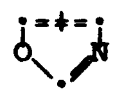 ,

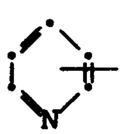 , 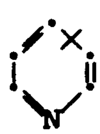 , 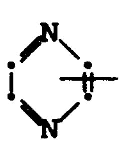 or any of the above heterocyclic structures wherein two adjacent H's are replaced with 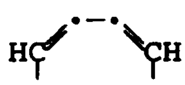 or 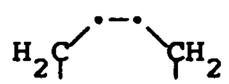 .

In a specific embodiment, the present process is employed to make 5-[2-(ethylthio)-propyl]-1,3-cyclohexanedione of the formula

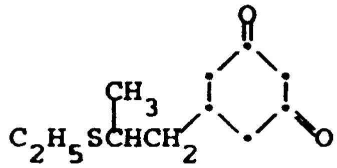

(II)

which is an intermediate in the preparation of the herbicide of the formula

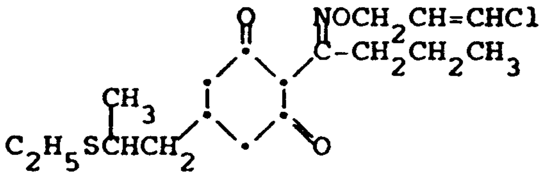

sold under the tradename SELECTONE. This herbicide is prepared from the said intermediate by the reaction sequence

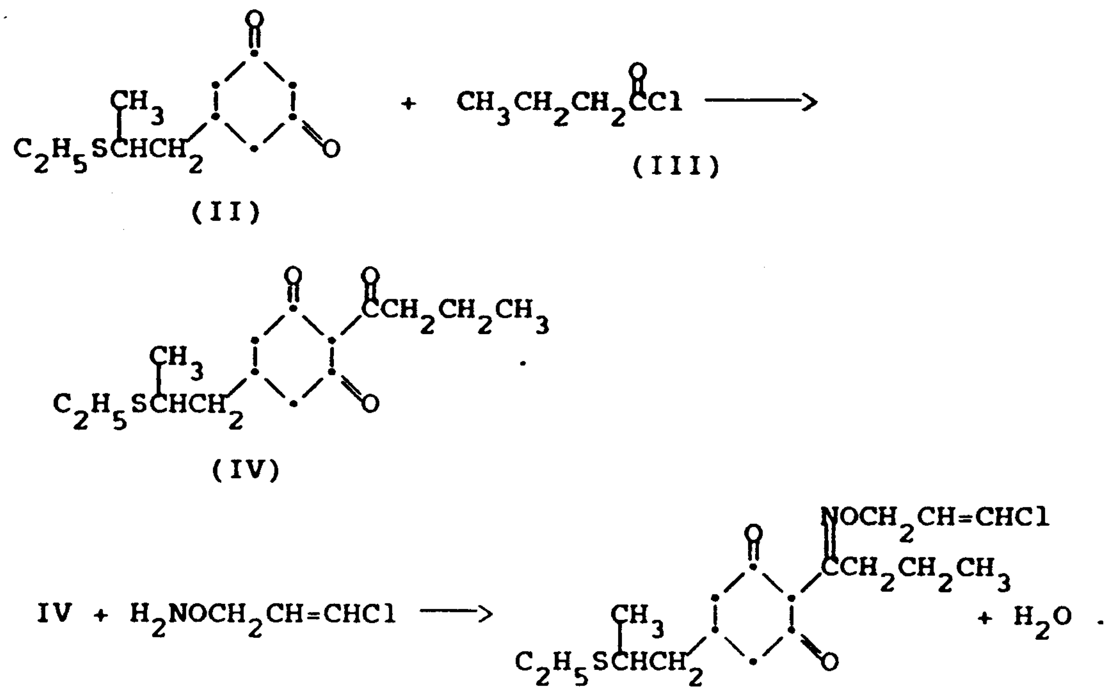

(II) (III)

(IV)

A reported route to the aforesaid intermediate 5-[2-(ethylthio)-propyl]-1,3-cyclohexanedione (II) involves the condensation of acetoacetic acid and 3-ethylthiobutyraldehyde, followed by Michael addition with diethyl malonate, hydrolysis, and then decarboxylation. The economics of this route are poor from the standpoint of yield and the high cost of diethyl malonate. By contrast, the present invention gives good yields and obviates the need for this expensive reagent.

The following examples will further illustrate the invention.

EXAMPLE 1 - Preparation of 4-Carboethoxy-5-[2-(ethylthio)-propyl]-1,3-cyclohexanedione

The unsaturated ester of the formula $CH_3CH_2S(CH_3)CHCH_2CH=CHCOOCH_3$ (1.0 g, 5.3 mmole) was added to a solution of ethyl acetoacetate (0.68 g, 5.3 mmole) and sodium metal (0.36 g, 16.9 mmole) in 20 mL of dry ethanol and heated to reflux for 18 hours. After cooling, the ethanol was evaporated and partitioned between $H_2O$ and $CH_2Cl_2$. The aqueous product layer was acidified with HCl to pH=l and extracted with $CH_2Cl_2$. This organic layer was dried over sodium sulfate and evaporated to give 0.88 g

(57.8%) of product as a yellow oil which was partially crystalline. The target product was identified by IR and NMR.

EXAMPLE 2 - 5-[2-(Ethylthio)-propyl]-1,3-cyclohexanedione

The ester product of Example 1 (0.70 g, 2.4 mmole) was dissolved in 15 ml of methanol and 1 ml of 2N NaOH and heated to reflux for 2-1/2 hours. The mixture was maintained at room temperature overnight. Methanol was evaporated, and the residue partitioned between about 30 ml of toluene and about 30 ml of aqueous HCl. The toluene extract was dried over sodium sulfate and evaporated to give 0.35 g (66.8%) of product as a yellow solid which was identical with an authentic sample. This target product was further identified by NMR.

The invention has been described in detail with particular reference to preferred embodiments thereof, but it will be understood that variations and modifications will be effected within the scope of the invention.

## Claims

1. The process comprising reacting an acetoacetic ester of the formula $R\text{-}OOCCH_2COCH_3$ with an unsaturated ester of the formula $R^1R^2C=CHCOOR^3$ in the presence of basic catalyst to give a product of the formula

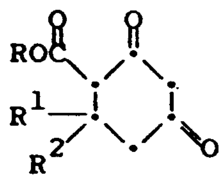

wherein: R is alkyl of 1-6 carbons: $R^1$ and $R^2$ are each selected independently from H, alkyl, cycloalkyl, alkyl substituted cycloalkyl, aryl, substituted aryl, heterocyclic, or substituted heterocyclic, with the provision that at least one of $R^1$ and $R^2$ is an alkylthioalkyl group, wherein the alkyl groups or moieties are 1-18 carbons; and $R^3$ is alkyl of 1-6 carbons or cycloalkyl.

2. The process of claim 1 wherein the alkyl groups or moieties are 1-6 carbons.

3. The process of claim 1 wherein R is $CH_2CH_3$, $R^1$ is H, $R^2$ is $CH_3CH_2S(CH_3)CHCH_2$-,and $R^3$ is $CH_3$.

4. The process of claim 1 wherein the catalyst is an alcoholic group IA or IIA metal alkoxide of an aliphatic alcohol having 1-6 carbon atoms.

5. The process of claim 1 wherein the catalyst if prepared in situ by reacting group IA or IIA metal with a $C_1$-$6_6$ alcohol.

6. The process of any of claim 1 to 5 wherein the catalyst is sodium ethoxide, the $C_1$-$C_6$ alcohol is ethanol, the molar ratio of sodium ethoxide to total moles of reactants is from about 0.10 to about 10.0, the molar ratio of acetoacetic ester to unsaturated ester is from about 0.8 to about 1.5, the weight ratio of total reactants to ethanol is from about 0.01 to about 1.2, and the reaction temperature is from about 22° C to about 78° C.

7. The process of claim 6 wherein the catalyst is sodium ethoxide, the molar ratio of sodium ethoxide to total moles of reactants is from about 1.0 to about 4.0, the molar ratio of acetoacetic ester to unsaturated ester is from about 1.0 to about 1.1, the weight ratio of total reactants to ethanol is from about 0.08 to about 1.0, and the reaction temperature is from about 70° C to about 78° C.

## Revendications

1. Procédé comprenant la réaction d'un ester acétoacétique de formule $R\text{-}OOCCH_2COCH_3$ avec un ester insaturé de formule $R^1R^2C=CHCOOR^3$ en présence d'un catalyseur basique pour former un produit de formule

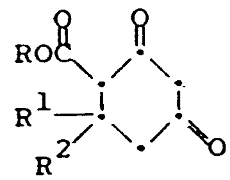

où

R est alkyle de 1-6 atomes de carbone ; $R^1$ et $R^2$ sont choisis indépendamment parmi H, alkyle, cycloalkyle, alkyl-cycloalkyle, aryle, aryle substitué, hétérocyclique ou hétérocyclique substitué, avec la condition qu'au moins l'un des $R^1$ et $R^2$ est un groupe alkylthioalkyle, dans lequel les groupes ou restes alkyle ont 1-18 atomes de carbone ; et $R^3$ est alkyle de 1-6 atomes de carbone ou cycloalkyle.

**2.** Procédé de la revendication 1, où les groupes ou restes alkyle ont 1-6 atomes de carbone.

**3.** Procédé de la revendication 1, où R est $CH_2CH_3$, $R^1$ est H, $R^2$ est $CH_3CH_2S(CH_3)CHCH_2$- et $R^3$ est $CH_3$.

**4.** Procédé de la revendication, 1, où le catalyseur est un alkoxyde alcoolique d'un métal des groupes IA ou IIA et d'un alcool aliphatique ayant 1-6 atomes de carbone.

**5.** Procédé de la revendication 1, où le catalyseur est préparé in situ en faisant réagir un métal des groupes IA ou IIA avec un alcool en $C_1$-$C_6$.

**6.** Procédé de l'une des revendications 1 à 5, où le catalyseur est l'éthoxyde de sodium, l'alcool en $C_1$-$C_6$ est l'éthanol, le rapport molaire de l'éthoxyde de sodium au total des réactifs est de 0,10 à 10,0, environ, le rapport molaire de l'ester acétoacétique à l'ester insaturé est de 0,8 à 1,5, environ, le rapport en masse du total des réactifs à l'éthanol est de 0,01 à 1,2, environ et la température de réaction est de 22 à 78 °C, environ.

**7.** Procédé de la revendication 6, où le catalyseur est l'éthoxyde de sodium, le rapport molaire de l'éthoxyde de sodium au total des réactifs est de 1,0 à 4,0 environ, le rapport molaire de l'ester acétoacétique à l'ester insaturé est de 1,0 à 1,1, environ, le rapport en masse du total des réactifs à l'éthanol est de 0,08 à 1,0, environ et la température de réaction est de 70 °C à 78 °C environ.

**Patentansprüche**

**1.** Verfahren, bei dem man einen Acetoessigester der Formel $R\text{-}OOCCH_2COCH_3$ mit einem ungesättigten Ester der Formel $R^1R^2C\text{-}CHCOOR^3$ in Gegenwart eines basischen Katalysators unter Bildung eines Produktes der Formel

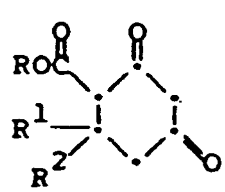

umsetzt, in der bedeuten: R gleich Alkyl mit 1-6 Kohlenstoffatomen; $R^1$ und $R^2$ jeweils unabhängig voneinander H, Alkyl, Cycloalkyl, Alkyl-substituiertes Cycloalkyl, Aryl, substituiertes Aryl, einen heterocyclischen Rest oder einen substituierten heterocyclischen Rest, wobei gilt, daß mindestens einer der Reste $R^1$ und $R^2$ eine Alkylthioalkylgruppe ist und die Alkylgruppen oder Reste 1-18 Kohlenstoffatome aufweisen; und $R^3$ für Alkyl mit 1-6 Kohlenstoffatomen oder Cycloalkyl steht.

**2.** Verfahren nach Anspruch 1, in dem die Alkylgruppen oder Reste 1-6 Kohlenstoffatome aufweisen.

3. Verfahren nach Anspruch 1, in dem R für $-CH_2CH_3$ steht, $R^1$ gleich H ist, $R^2$ für $CH_3CH_2S(CH_3)CHCH_2$-steht und $R^3$ gleich $-CH_3$ ist.

4. Verfahren nach Anspruch 1, in dem der Katalysator ein Alkoxid eines Metalles aus der Gruppe IA oder IIA eines aliphatischen Alkohols mit 1-6 Kohlenstoffatomen ist.

5. Verfahren nach Anspruch 1, in dem der Katalysator in situ hergestellt ist durch Umsetzung eines Metalles der Gruppe IA oder IIA mit einem $C_1$-$C_6$-Alkohol.

6. Verfahren nach einem der Ansprüche 1 bis 5, in dem der Katalysator aus Natriumethoxid besteht, der $C_1$-$C_6$-Alkohol Ethanol ist, das Molverhältnis von Natriumethoxid zu den Gesamtmolen an Reaktionskomponenten bei etwa 0,10 bis etwa 10,0 liegt, das Molverhältnis von Acetoessigester zu ungesättigtem Ester etwa 0,8 bis 1,5 beträgt, das Gewichtsverhältnis der gesamten Reaktionskomponenten zu Ethanol bei etwa 0,01 bis etwa 1,2 liegt und die Reaktionstemperatur etwa 22°C bis etwa 78°C beträgt.

7. Verfahren nach Anspruch 6, in dem der Katalysator Natriumethoxid ist, das Molverhältnis von Natriumethoxid zu den Gesamtmolen an Reaktionskomponenten bei etwa 1,0 bis etwa 4,0 liegt, das Molverhältnis von Acetoessigester zu ungesättigtem Ester bei etwa 1,0 bis etwa 1,1 liegt, das Gewichtsverhältnis der gesamten Reaktionskomponenten zu Ethanol etwa 0,08 bis etwa 1,0 beträgt und die Reaktionstemperatur bei etwa 70°C bis etwa 78°C liegt.